(19) Europäisches Patentamt
European Patent Office
Office européen des brevets

(11) EP 4 716 091 A1

(12) EUROPÄISCHE PATENTANMELDUNG

(43) Veröffentlichungstag:
25.03.2026 Patentblatt 2026/13

(21) Anmeldenummer: 24201968.5

(22) Anmeldetag: 23.09.2024

(51) Internationale Patentklassifikation (IPC):
H02P 21/14 (2016.01)    H02P 23/14 (2006.01)
H02P 29/024 (2016.01)    H02P 29/60 (2016.01)
A61M 60/538 (2021.01)    H02P 6/182 (2016.01)

(52) Gemeinsame Patentklassifikation (CPC):
H02P 23/14; A61M 60/178; A61M 60/232;
A61M 60/422; A61M 60/538; F04D 13/06;
F04D 15/0088; H02P 21/14; H02P 29/024;
H02P 29/0243; H02P 29/025; H02P 29/60;
H02P 6/182

(84) Benannte Vertragsstaaten:
AL AT BE BG CH CY CZ DE DK EE ES FI FR GB
GR HR HU IE IS IT LI LT LU LV MC ME MK MT NL
NO PL PT RO RS SE SI SK SM TR
Benannte Erstreckungsstaaten:
BA
Benannte Validierungsstaaten:
GE KH MA MD TN

(71) Anmelder: Berlin Heart GmbH
12247 Berlin (DE)

(72) Erfinder:
• BYKOV, Valentin
12247 Berlin (DE)
• KIESNER, Matthias
15834 Rangsdorf (DE)
• PETERS, Oliver
12247 Berlin (DE)
• JAHNE, Helmut
12247 Berlin (DE)

(74) Vertreter: Pfenning, Meinig & Partner mbB
Patent- und Rechtsanwälte
Joachimsthaler Straße 10-12
10719 Berlin (DE)

(54) STEUEREINHEIT FÜR EINE ROTATIONSFLUIDPUMPE, PUMPENSYSTEM UND VERFAHREN

(57) Die Anmeldung betrifft Steuereinheiten (300) für eine Rotationsfluidpumpe (200), insbesondere eine Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221), umfasst. Eine beispielhafte Steuereinheit (300) ist eingerichtet zum: Erzeugen eines Steuersignals für mindestens eine der Mehrzahl von Motorspulen (221), wobei das Steuersignal mit einem Modulationssignal beaufschlagt wird, Erfassen mindestens eines Messsignals, entsprechend einem durch die mindestens eine der Mehrzahl von Motorspulen (221) fließenden Strom und/oder einer an der mindestens einen der Mehrzahl von Motorspulen (221) anliegenden Spannung, Demodulieren des mindestens einen Messsignals zum Bestimmen mindestens eines veränderlichen charakteristischen elektrischen Widerstands einer elektrischen Anordnung, umfassend die mindestens eine der Mehrzahl von Motorspulen (221) und eine zum Verbinden der mindestens einen der Mehrzahl von Motorspulen (221) mit der Steuereinheit (300) eingerichtete Zuleitung. Daneben betrifft die Anmeldung Pumpensysteme (100) sowie Verfahren zum Bestimmen mindestens eines veränderlichen charakteristischen elektrischen Widerstands einer elektrischen Anordnung einer Rotationsfluidpumpe (200).

FIG. 1

**Beschreibung**

Die Anmeldung betrifft Steuereinheiten für Rotationsfluidpumpen, Pumpensysteme mit Rotationsfluidpumpen sowie Verfahren zum Bestimmen mindestens eines veränderlichen charakteristischen elektrischen Widerstands einer elektrischen Anordnung einer Rotationsfluidpumpe. Der Gegenstand der Anmeldung ist insbesondere im Gebiet der Herzunterstützungsvorrichtungen und -systeme anwendbar, wobei es sich bei den Rotationsfluidpumpen insbesondere um Blutpumpen handeln kann.

Aus dem Stand der Technik sind Rotationsfluidpumpen, insbesondere als Blutpumpen ausgelegte Rotationsfluidpumpen, etwa für den Einsatz als Herzunterstützungsvorrichtung (kurz VAD für "ventricular assist device"), sowie entsprechende Steuereinheiten und Steuerverfahren bekannt. Solche Pumpen umfassen typischerweise einen (in Bezug auf einen Installations- oder Implantationsort der Pumpe im Wesentlichen ortsfesten) Stator sowie einen zum Fördern von Fluid bezüglich des Stators um eine Rotationsachse rotierbaren Rotor, wobei Rotor und Stator eine Elektromotoranordnung (im Folgenden auch kurz als Motor bezeichnet) bilden. Zum erwünschten Regeln der Rotation des Rotors ist die Kenntnis einer rotatorischen Position des Rotors erforderlich. Auch kann die Kenntnis einer translatorischen Position des Rotors erforderlich sein, beispielsweise für eine Positionsregelung für einen in Bezug auf den Stator in mindestens einem Freiheitsgrad aktiv magnetisch gelagerten Rotor (wobei der Rotor insbesondere berührungsfrei, also "schwebend", gelagert wird). Bei der Konstruktion und im Betrieb solcher Pumpen sind verschiedene Anforderungen zu berücksichtigen und gegeneinander abzuwägen sowie Nachteile zu vermeiden oder zu verringern. Beispielsweise soll eine Rotationsfluidpumpe möglichst kompakt, leicht, robust sowie im Betrieb sicher, effizient und zuverlässig sein.

Werden, wie beispielhaft aus dem Stand der Technik bekannt, gesonderte Sensoren zum Erfassen der rotatorischen und/oder translatorischen Position des Rotors vorgesehen, so können sich dadurch etwa Baugröße, Masse, Komplexität und/oder Energieverbrauch der Pumpe erhöhen.

Alternativ ist eine Positionsbestimmung auf Grundlage der Bestimmung von positionsabhängigen Induktionsspannungen (sogenannte "back electromotive force", BEMF), die durch Magnete des Rotors in Motorspulen des Stators erzeugt werden, vorgeschlagen worden. Da diese Bestimmung nur indirekt - insbesondere basierend auf einer Messung der Ströme und Spannungen auf allen Motorleitungen sowie auf einem elektrischen Modell des gebildeten Elektromotors - erfolgen kann, ist hierfür eine möglichst genaue Kenntnis von Modellparametern des elektrischen Modells erforderlich.

Solche Modellparameter können etwa durch Kalibration, beispielsweise durch einmalige Kalibration vor Installation bzw. Implantation der Pumpe, bestimmt werden. Jedoch können solche Modellparameter zeitlich veränderlich (beispielweise abhängig von Betriebszuständen und/oder Alterungserscheinungen der Pumpe und/oder anderer Teile des Pumpensystems) sein, was die Genauigkeit beim Bestimmen der Induktionsspannungen und somit die Effizienz, Sicherheit und/oder Zuverlässigkeit im Betrieb der Pumpe beeinträchtigen kann. Beispielsweise kann der Fall auftreten, dass das Bestimmen der Induktionsspannung auf Grundlage fester (etwa durch einmalige Kalibrierung bestimmter) Modellparameter nicht ausreicht für eine schwebende Lagerung des Rotors, bei der insbesondere zugleich noch ein Impulseintrag durch externe Schläge auf die Pumpe über eine Spanne von Betriebszuständen (etwa bei verschiedenen Temperaturen der Pumpe) ausgeregelt werden kann.

Dementsprechend liegt der Anmeldung die Aufgabe zugrunde, Lösungen hinsichtlich der Konstruktion, des Betriebs und/oder der Ansteuerung von Rotationsfluidpumpen bereitzustellen, die die genannten Anforderungen wenigstens zum Teil erfüllen und/oder die genannten Nachteile wenigstens zum Teil vermeiden oder verringern.

Zur Lösung der Aufgabe werden die Gegenstände der unabhängigen Ansprüche vorgeschlagen. Bevorzugte Weiterbildungen und optionale Merkmale ergeben sich mit den Merkmalen der abhängigen Ansprüche.

Vorgeschlagen wird zunächst eine Steuereinheit für eine Rotationsfluidpumpe, wobei die Rotationsfluidpumpe einen zum Fördern von Fluid um eine Rotationsachse rotierbaren Rotor sowie einen Stator mit einer Mehrzahl von Motorspulen, umfasst. Die Motorspulen sind vorzugsweise eingerichtet zum Erzeugen eines bezüglich der Rotationsachse auf den Rotor wirkenden Drehmoments. Die Rotationsfluidpumpe kann als Blutpumpe, insbesondere als VAD, ausgelegt sein, wobei das geförderte Fluid Blut ist. In diesem Fall kann die Blutpumpe implantierbar sein, jedoch sind auch wenigstens teilweise extrakorporale Ausführungen denkbar. Die Steuereinheit kann eine externe (im Falle der implantierbaren Blutpumpe insbesondere extrakorporale), mit der Blutpumpe mittels einer Driveline verbindbare oder verbundene Steuereinheit ein. Die Steuereinheit kann jedoch auch ganz oder teilweise in die Blutpumpe integriert und/oder mit dieser implantierbar sein. Es kann auch vorgesehen sein, dass die Steuereinheit sowohl an der Blutpumpe angeordnete als auch extern/extrakorporal angeordnete Teile umfasst.

Die Rotationsfluidpumpe ist nicht auf eine Blutpumpe beschränkt und kann alternativ beispielsweise zum Fördern von Wasser, Öl oder einem anderen Fluid vorgesehen sein.

Der Stator kann an einem Gehäuse der Pumpe angeordnet sein und/oder dieses bilden. Das Gehäuse umfasst vorzugsweise einen Fluideinlass und einen Fluidauslass, die im Falle der Blutpumpe mit jeweiligen Blutgefäßen und/oder einem Herzen fluidisch verbindbar sind.

Die Steuereinheit ist eingerichtet zum Erfassen mindestens eines Messsignals, entsprechend einem durch mindestens eine der Mehrzahl von Motorspulen fließenden Strom und/oder einer an der mindestens einen der Mehrzahl von Motorspulen anliegenden Spannung.

Die Steuereinheit kann ferner eingerichtet sein zum:

Erzeugen eines Steuersignals für die mindestens eine der Mehrzahl von Motorspulen, wobei das Steuersignal mit einem Modulationssignal beaufschlagt wird,

Demodulieren des mindestens einen Messsignals zum Bestimmen mindestens eines veränderlichen charakteristischen elektrischen Widerstands einer elektrischen Anordnung, umfassend die mindestens eine der Mehrzahl von Motorspulen und eine zum Verbinden der mindestens einen der Mehrzahl von Motorspulen mit der Steuereinheit eingerichtete Zuleitung.

Die Erfinder haben festgestellt, dass die Kenntnis des veränderlichen charakteristischen elektrischen Widerstands der genannten Art, im Folgenden mitunter auch kurz als Phasenwiderstand bezeichnet, entscheidend ist für ein Bestimmen der Induktionsspannungen mit hoher Genauigkeit, insbesondere mit hinreichender Genauigkeit für eine zuverlässige Regelung der Rotation und/oder Position des Rotors auf Grundlage der Positionsabhängigkeit der Induktionsspannungen. Das Messprinzip mit Beaufschlagen des Steuersignals mit dem Modulationssignal und Demodulieren des mindestens einen Messsignals ermöglicht eine genaue Bestimmung des Phasenwiderstandes, wobei verschiedene Störeinflüsse - einschließlich Variation der Induktivität, Gegeninduktion und/oder Wirbelstromverluste im Betrieb des Motors -unterdrückt werden. Die vorgeschlagene Steuereinheit ermöglicht es daher gemäß den obenstehenden Überlegungen, eine besonders kompakte, leichte und robuste Rotationsfluidpumpe vorzusehen und diese besonders sicher, effizient und zuverlässig zu betreiben. Das Beaufschlagen des Steuersignals mit dem Modulationssignal und Demodulieren des mindestens einen Messsignals eignet sich insbesondere für ein genaues Bestimmen des Phasenwiderstands bzw. von Änderungen des Phasenwiderstands über Zeitskalen, die erheblich länger sind als eine Rotationsdauer des Rotors und/oder ein Inverses einer Drehfelddrehzahl (wie weiter unten definiert) im Betrieb (beispielsweise über ca. 1 bis 10 s bei einer Drehfelddrehzahl von beispielsweise 0,1 bis 1 kHz).

Die Steuereinheit kann zusätzlich oder alternativ eingerichtet sein zum:

Bestimmen, basierend auf dem mindestens einen Messsignal und einem thermischen Modell, einer Änderung einer Temperatur wenigstens eines Teils der Rotationsfluidpumpe, umfassend die mindestens eine der Mehrzahl von Motorspulen, über ein Messintervall; und

Bestimmen, basierend auf der bestimmten Änderung der Temperatur, eines Wertes oder einer Änderung mindestens eines veränderlichen charakteristischen elektrischen Widerstands einer elektrischen Anordnung, umfassend die mindestens eine der Mehrzahl von Motorspulen und eine zum Verbinden der mindestens einen der Mehrzahl von Motorspulen mit der Steuereinheit eingerichtete Zuleitung.

Auch mittels dieser weiteren Methode zum Bestimmen des Phasenwiderstands wird es entsprechend den obigen Ausführungen ermöglicht, eine besonders kompakte, leichte und robuste Rotationsfluidpumpe vorzusehen und diese besonders sicher, effizient und zuverlässig zu betreiben. Das Bestimmen der Temperaturänderung basierend auf dem mindestens einen Messsignal und dem thermischen Modell eignet sich insbesondere für ein genaues Bestimmen des Phasenwiderstands bzw. von Änderungen des Phasenwiderstands über kürzere Zeitskalen als die oben für die demodulationsbasierte Methode genannten Zeitskalen; solche Änderungen können etwa durch plötzlichen Impulseintrag, etwa durch externe Schläge oder Beschleunigungen der Pumpe, verursacht werden. Durch einen solchen Impulseintrag kann - bedingt durch die erforderlichen Regelvorgänge - die Temperatur der Motorspulen kurzfristig signifikant ansteigen und sich damit der Phasenwiderstand entsprechend ändern. Auch bei einem Startvorgang des Motors, der bei einem magnetisch gelagerten und entsprechend vorgespannten Rotor mit einem Ablösen des Rotors von einer Gehäusewand einhergeht, kann aufgrund der dafür erforderlichen hohen Leistung eine schnelle Temperaturerhöhung erfolgen.

Die Steuereinheit kann dazu eingerichtet sein, den mindestens einen veränderlichen charakteristischen elektrischen Widerstand wiederholt für jeweils ein erstes Messintervall durch Demodulieren des mindestens einen Messsignals zu bestimmen, insbesondere in einem Normalbetrieb der Rotationsfluidpumpe. Der Normalbetrieb stellt dabei einen normalen Betriebszustand ohne momentan erhöhten Leistungseintrag dar. Die Steuereinheit kann dazu eingerichtet sein, eine Änderung des mindestens einen veränderlichen charakteristischen elektrischen Widerstands über ein zweites Messintervall basierend auf der bestimmten Änderung der Temperatur zu bestimmen, beispielsweise in Antwort auf ein Erfassen eines gegenüber dem Normalbetrieb erhöhten Leistungseintrags in die Rotationsfluidpumpe (im Folgenden auch als Ausnahmebetrieb bezeichnet). Ein wiederholtes Bestimmen basierend auf der Temperaturänderung über das zweite Messintervall kann auch im Normalbetrieb erfolgen. Das zweite Messintervall ist vorzugsweise kürzer als das erste Messintervall. Besonders vorteilhaft können die beschriebenen Messungen über das erste Messintervall durch Demodulieren und über das zweite Messintervall basierend auf der Temperaturänderung miteinander kombiniert werden. Somit ist eine Bestimmung des Phasenwiderstands bzw. seinerÄnderungen mit guter Genauigkeit über sowohl kürzere als auch längere Zeitskalen möglich, wodurch auf langsamere und schnellere Änderungen entsprechend reagiert werden kann.

Das Messsignal umfasst vorzugsweise - als vektorielles Messsignal - Komponenten entsprechend den durch jede der Mehrzahl von Motorspulen fließenden Ströme und den an jeder der Mehrzahl von Motorspulen anliegenden Spannungen. Die Steuereinheit kann zum sequentiellen und/oder gleichzeitigen Erfassen der genannten Komponenten eingerichtet sein.

Es soll darauf hingewiesen werden, dass das als Modulationssignal bezeichnete Signal ein in Amplitude und/oder Frequenz und/oder Phase konstantes Signal sein kann, das also für sich genommen keine aufmodulierte Information enthält. Eine Amplituden- und Phasenmodulation des mit dem Modulationssignal beaufschlagten Steuersignals ergibt sich dann durch den Motor selbst, der also als Modulator wirkt. Das erfassbare Messsignal ist somit ein moduliertes Signal, dem durch Demodulieren Information insbesondere über den Phasenwiderstand in der beschriebenen Weise entnehmbar ist.

Es kann vorgesehen sein, dass der Stator eine Mehrzahl von Motorphasen, umfassend jeweils mindestens eine der Mehrzahl von Motorspulen, umfasst. Die Steuereinheit kann dann dazu eingerichtet sein,

nacheinander eine Mehrzahl von Modulationszuständen vorzugeben, wobei in jedem Modulationszustand ein jeweiliger Satz von Motorphasen gegenphasig oder phasenverschoben mit dem Modulationssignal beaufschlagt wird und

wobei das Messsignal im jeweiligen Modulationszustand an dem jeweils mit dem Modulationssignal beaufschlagten Satz von Motorphasen abgegriffen wird.

Auf diese Weise können alle relevanten Komponenten des Messsignals zum Bestimmen aller relevanten Phasenwiderstände des Motors erfasst werden.

Eine Modulationsfrequenz des Modulationssignals kann weniger als eine Drehfelddrehzahl eines die Rotation des des Rotors verursachenden Drehfelds, vorzugsweise weniger als 50 Hz, betragen. Die Drehfelddrehzahl ist dabei insbesondere als Produkt aus einer Rotationsfrequenz des Rotors und einer Anzahl von Polpaaren des Rotors darstellbar. Durch eine solche Wahl der Modulationsfrequenz kann - insbesondere in Verbindung mit hinreichend geringer Amplitude des Modulationssignals - ein Störeinfluss des Modulationssignals auf das Steuersignal vermieden bzw. minimiert werden. Die Drehfelddrehzahl kann beispielweise im Bereich von 0,1 bis 1 kHz liegen.

Zumindest ein Anteil des erfassten Messsignals und/oder eine basierend auf dem Messsignal bestimmte Größe (insbesondere ein Spannungs- und/oder Stromwert) kann zum Bestimmen des mindestens einen veränderlichen charakteristischen elektrischen Widerstands zeitlich gemittelt und/oder kumuliert werden, insbesondere über ein Intervall von mindestens 1 s und/oder höchstens 10 s. Auf diese Weise kann ein erhöhter Signal-Rauschabstand und eine entsprechend genaue Messung des Phasenwiderstandes erzielt werden. Das Intervall kann zur Laufzeit an die momentanen Anforderungen angepasst werden; beispielsweise kann eine schnelle, kurzzeitige Messung mit reduziertem Intervall bei erhöhter Modulationsamplitude durchgeführt werden.

Die Steuereinheit kann dazu eingerichtet sein, das Steuersignal unter Verwendung von Pulsbreitenmodulation zu erzeugen. Die Steuereinheit kann dazu eingerichtet sein, das Steuersignal mit dem Modulationssignal durch Variation der Pulsbreitenmodulation, insbesondere durch Aufaddieren einer Signalform, insbesondere eines Rechtecksignals, zu beaufschlagen. Die Steuereinheit kann dazu eingerichtet sein, einen durch das Beaufschlagen des Steuersignals mit dem Modulationssignal verursachten Modulationsanteil des Messsignals mittels Demodulation, insbesondere Synchrondemodulation, zu erfassen.

Die Zuleitung bzw. jede der zum Verbinden einer jeweiligen Motorspule mit der Steuereinheit eingerichtete Zuleitung umfasst typischerweise wenigstens eine leitende Ader (optional mehrere leitende Adern) und vorzugsweise wenigstens einen Kontakt, etwa einen Steckkontakt, zum Verbinden der Ader mit der Pumpe und/oder der Steuereinheit. Die Kontakte können fest und/oder lösbar ausgeführt sein.

Der Rotor kann in Bezug auf den Stator in wenigstens einem Freiheitsgrad, etwa entlang der Rotationsachse, magnetisch, insbesondere aktiv magnetisch, gelagert sein. Der Rotor kann vollmagnetisch, d. h. in allen Freiheitsgraden magnetisch gelagert sein. Für eine vollmagnetische Lagerung sind beispielsweise mehrere getrennte Magnetbaugruppen (Spulen und/oder Permanentmagnete) sowohl im Stator als auch im Rotor zur Lagerung in jeweiligen Freiheitsgraden vorgesehen worden. Die Motorspulen selbst können - neben dem Erzeugen des Drehmoments für die Rotation - auch zur magnetischen Lagerung des Rotors in wenigstens einem Freiheitsgrad, insbesondere entlang der Rotationsachse, vorgesehen sein. Alternativ oder zusätzlich können gesonderte Spulen oder Spulengruppen für die magnetische Lagerung vorgesehen sein.

Die Steuereinheit kann dazu eingerichtet sein, die Rotation des Rotors und/oder eine rotatorische und/oder translatorische Position des Rotors basierend auf dem Messsignal und einem Modell wenigstens eines Teils der Rotationsfluidpumpe wenigstens zu erfassen, vorzugsweise auch zu regeln, wobei das Modell den mindestens einen veränderlichen charakteristischen elektrischen Widerstand umfasst. Als Regeln wird hier ein Regeln mit Rückkopplung (also "closed-loop control") bezeichnet. Die Steuereinheit kann zur entsprechenden Ansteuerung der Motorspulen, etwa mittels Blockkommutierung, Sinuskommutierung, Raumzeigermodulation und/oder Vektorregelung (field-oriented control, FOC), eingerichtet sein. Beispielsweise kann bei Ansteuerung mittels einer Vektorregelung unter Verwendung eines durch Park-Transformation erhaltenen dq-Systems die Rotation des Rotors über den Drehmomentstrom ($I_q$), eine Axialkraft zur Positionsregelung entlang der Rotationsachse über die Feldkomponente ($I_d$) geregelt werden.

Die Steuereinheit kann dazu eingerichtet sein, die Änderung der Temperatur und/oder den Wert oder die Änderung des Phasenwiderstands basierend auf einem auf Grundlage des Messsignals bestimmten Leistungseintrag in die Rotationsfluidpumpe und/oder basierend auf einer geschätzten thermischen Leistungsabgabe der Rotationsfluidpumpe zu bestimmen. Der Leistungseintrag und/oder die Leistungsabgabe kann insbesondere als Input für das thermische Modell verwendet werden.

Die Steuereinheit kann dazu eingerichtet sein, basierend auf dem mindestens einen veränderlichen charakteristischen elektrischen Widerstand einen Verbindungszustand zwischen der Rotationsfluidpumpe und der Steuereinheit und/oder einen Defektzustand der Rotationsfluidpumpe und/oder der Zuleitung zu erfassen. Durch Herstellen bzw. Lösen der Verbindung (also Ändern des Verbindungszustands) oder Defekte (wie beispielsweise Aderbruch in der Pumpe und/oder der Zuleitung) kommt es zu Änderungen des Phasenwiderstands, die mit den vorgeschlagenen Methoden erfasst werden können. Das Erfassen des Verbindungszustands kann beispielsweise einen Pull-Up/Down-Widerstand in der Steckverbindung ersetzen und somit den Hardwareaufwand verringern. Zudem werden in diesem Fall dieselben Leitungen und Sensoren verwendet wie für den Betrieb der Pumpe, so dass dieses Erfassen gegenüber einem Ansatz mit separaten Pins die Zuverlässigkeit verbessern kann. Das Erfassen von Defektzuständen kann die Sicherheit der Pumpe verbessern (etwa kann in Antwort auf ein solches Erfassen eine Warnung ausgegeben und/oder auf einen alternativen Betriebsmodus umgeschaltet werden).

Vorgeschlagen wird auch ein Pumpensystem, umfassend

eine Rotationsfluidpumpe, umfassend einen zum Fördern von Fluid um eine Rotationsachse rotierbaren Rotor sowie einen Stator mit einer Mehrzahl von Motorspulen, und
eine Steuereinheit der vorgeschlagenen Art.

In dem vorgeschlagenen Pumpensystem entfaltet die Steuereinheit in offensichtlicher Weise ihre oben genannten und weiteren Wirkungen und Vorteile.

Entsprechende Wirkungen und Vorteile entfalten sich auch in einem Verfahren zum Bestimmen mindestens eines veränderlichen charakteristischen elektrischen Widerstands einer elektrischen Anordnung einer Rotationsfluidpumpe der beschriebenen Art, wobei das Verfahren umfasst:
Erfassen mindestens eines Messsignals, entsprechend einem durch die mindestens eine der Mehrzahl von Motorspulen fließenden Strom und/oder einer an der mindestens einen der Mehrzahl von Motorspulen anliegenden Spannung,

Das Verfahren kann umfassen:

Erzeugen eines Steuersignals für mindestens eine der Mehrzahl von Motorspulen, wobei das Steuersignal mit einem Modulationssignal beaufschlagt wird,
Demodulieren des mindestens einen Messsignals zum Bestimmen des mindestens einen veränderlichen charakteristischen elektrischen Widerstands der elektrischen Anordnung, wobei die elektrische Anordnung die mindestens eine der Mehrzahl von Motorspulen und eine zum Verbinden der mindestens einen der Mehrzahl von Motorspulen mit der Steuereinheit eingerichtete Zuleitung umfasst.

Das Verfahren kann umfassen:

Bestimmen, basierend auf dem mindestens einen Messsignal und einem thermischen Modell, einer Änderung einer Temperatur wenigstens eines Teils der Rotationsfluidpumpe, umfassend die mindestens eine der Mehrzahl von Motorspulen, über ein Messintervall; und
Bestimmen, basierend auf der bestimmten Änderung der Temperatur, eines Wertes oder einer Änderung des mindestens einen veränderlichen charakteristischen elektrischen Widerstands der elektrischen Anordnung, umfassend die mindestens eine der Mehrzahl von Motorspulen und eine zum Verbinden der mindestens einen der Mehrzahl von Motorspulen mit der Steuereinheit eingerichtete Zuleitung.

Das Verfahren kann in leicht ersichtlicher Weise gemäß optionaler Merkmale der Steuereinheit und/oder des Pumpensystems weitergebildet werden und/oder umgekehrt.

Die nachfolgend beschriebenen Zeichnungen illustrieren Prinzipien und beispielhafte Ausführungsformen des Gegenstands der Anmeldung. Dabei zeigen, jeweils schematisch,

FIG. 1 ein Pumpensystem einschließlich einer Rotationsfluidpumpe in Längsschnittansicht,

FIG. 2 eine Schaltskizze eines Teils der Rotationsfluidpumpe nach FIG. 1,

FIG. 3 eine Prinzipskizze eines Verfahrens nach einem Beispiel,

FIG. 4 einen Teil des Pumpensystems nach FIG. 1,

FIG. 5 eine Prinzipskizze eines Verfahrens nach einem weiteren Beispiel.

Wiederkehrende und ähnliche Merkmale in den Zeichnungen sind mit identischen oder ähnlichen Bezugszeichen versehen. Diese können teilweise ausgelassen werden, wenn die entsprechenden Merkmale bereits in einer anderen Zeichnung gezeigt und im Zusammenhang mit dieser beschrieben werden oder wenn sie mit Bezug auf eine Zeichnung nicht erwähnt werden.

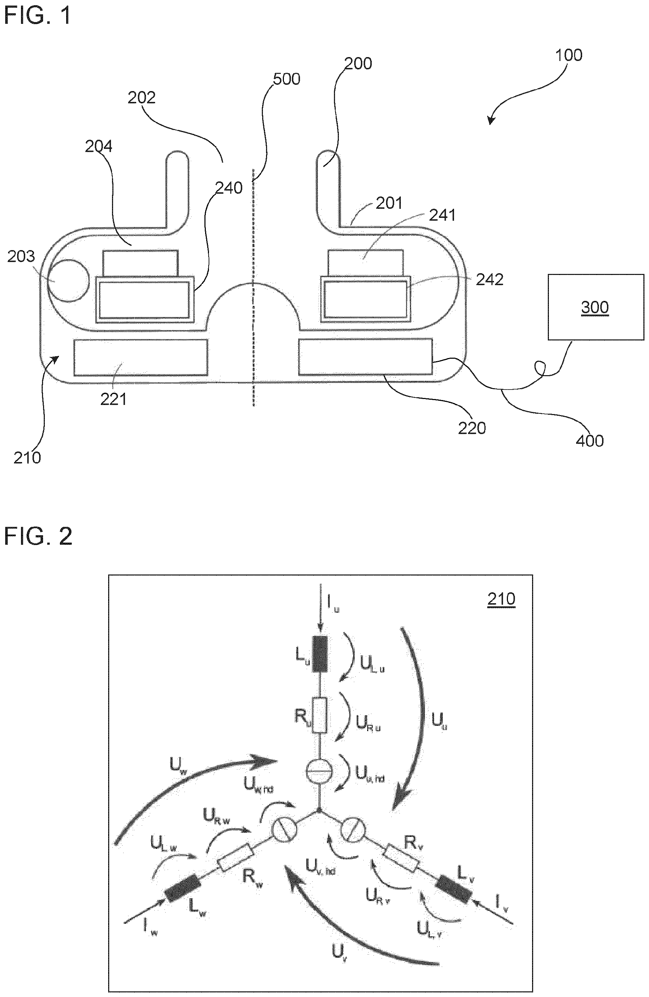

Das in FIG. 1 gezeigte Pumpensystem 100 umfasst eine Steuereinheit 300 und eine Rotationsfluidpumpe 200. Die Rotationsfluidpumpe 200 umfasst einen zum Fördern von Fluid um eine Rotationsachse 500 rotierbaren Rotor 240 sowie einen Stator 220 mit einer Mehrzahl von Motorspulen 221. Stator 220 und Rotor 240 bilden einen Motor 210, im gezeigten Beispiel einen Axialflussmotor. Der Anmeldungsgegenstand ist jedoch nicht auf Axialflussmotoren beschränkt und umfasst ferner beispielsweise Radialflussmotoren. Der Rotor 240 umfasst eine Rotormagnetanordnung 242, bestehend aus einem oder mehreren Permanentmagneten, die zum Wechselwirken mit den Motorspulen 221 des Stators 220 zum Erzeugen eines Drehmoments bezüglich der Rotationsache 500 eingerichtet ist.

Die Rotationsfluidpumpe 200 ist als implantierbare Blutpumpe, speziell als VAD bzw. teil eines VAD-Systems, ausgelegt, wobei das geförderte Fluid Blut ist. Auch wenigstens teilweise extrakorporale Ausführungen sind denkbar. Die Steuereinheit 300 ist eine externe (insbesondere extrakorporale), mit der Blutpumpe 200 mittels einer Driveline 400 verbindbare Steuereinheit 300. Die Steuereinheit 300 kann jedoch auch ganz oder teilweise in die Blutpumpe 200 integriert und/oder mit dieser implantierbar sein. Es kann auch vorgesehen sein, dass die Steuereinheit 300 sowohl an der Blutpumpe 200 angeordnete als auch extern/extrakorporal angeordnete Teile umfasst. Die Rotationsfluidpumpe 200 ist nicht auf eine Blutpumpe beschränkt und kann alternativ beispielsweise zum Fördern von Wasser, Öl oder einem anderen Fluid vorgesehen sein. Die beispielhaft gezeigte Pumpe 200 ist eine Zentrifugalpumpe, jedoch ist der Anmeldungsgegenstand nicht darauf beschränkt und umfasst etwa auch Radialpumpen oder Mischformen aus den genannten Pumpentypen.

Der Stator 220 ist an einem Gehäuse 201 der Pumpe angeordnet. Das Gehäuse 201 umfasst einen Fluideinlass 202 und einen Fluidauslass 203, die mit jeweiligen Blutgefäßen und/oder einem Herzen fluidisch verbindbar sind. Zum Fördern des Fluids vom Fluideinlass 202 zum Fluidauslass 203 umfasst der Rotor 240 eine Beschaufelung 241.

Der Rotor 240 ist innerhalb einer Kavität 204 des Gehäuses 201 berührungsfrei magnetisch gelagert. Hierzu umfassen Stator 220 und Rotor 240 geeignete Magnetanordnungen. Beispielsweise kann eine ringförmige Rotormagnetanordnung 242 im Rotor 240 zum Lagern des Rotors 240 entlang einer durch die Rotationsachse definierten Axialrichtung mit den Motorspulen 221 des Stators 220 wechselwirken, wobei die Motorspulen 221 zur Regelung der rotatorischen und/oder translatorischen Position des Rotors 240 ansteuerbar sind (etwa mittels Vektorregelung wie oben beschrieben).

Die Steuereinheit 300 ist eingerichtet zum Erfassen eines mehrkomponentigen (vektoriellen) Messsignals, umfassend Komponenten, die der durch jede der Mehrzahl von Motorspulen 221 fließenden Ströme und der an jeder der Mehrzahl von Motorspulen 221 anliegenden Spannungen entsprechen. Die Steuereinheit 200 kann zum sequentiellen und/oder gleichzeitigen Erfassen der genannten Komponenten eingerichtet sein.

Basierend auf dem Messsignal sind die positionsabhängigen Induktionsspannungen (BEMF-Spannungen), die durch die Rotormagnetanordnung 242 des Rotors 240 in den Motorspulen 221 des Stators 220 erzeugt werden, bestimmbar. Das Prinzip dieser Bestimmung kann anhand der in FIG. 2 gezeigten Schaltskizze erläutert werden. Die Schaltskizze zeigt beispielhaft einen Motor 210 mit drei Motorphasen u, v, w. Allgemeiner kann eine Mehrzahl von Motorphasen, umfassend jeweils mindestens eine der Mehrzahl von Motorspulen 221, vorgesehen sein.

Die Motorphasen u, v, w werden durch ihre Induktivitäten $L_u$, $L_v$, $L_w$ und ihre Widerstände $R_u$, $R_v$, $R_w$ dargestellt, an denen die jeweilige Spannungen $U_{L,u}$, $U_{R,u}$, $U_{L,v}$, $U_{R,v}$, $U_{L,w}$, $U_{R,w}$ abfallen. Jeweilige induzierte Spannungen werden als $U_{u,\,ind}$, $U_{v,\,ind}$, $U_{w,\,ind}$ bezeichnet. Die Widerstände $R_u$, $R_v$, $R_w$ entsprechen dabei den Widerständen jeweiliger Anordnungen, die jeweils eine der Mehrzahl von Motorspulen 221 und eine zum Verbinden der jeweiligen Motorspule 221 mit der Steuereinheit 300 eingerichtete Zuleitung umfassen (Phasenwiderstände).

Als Komponenten des Messsignals sind die durch die Motorphasen u, v, w insgesamt fließenden Ströme $I_u$, $I_v$, $I_w$ sowie die an den Motorphasen u, v, w insgesamt anliegenden Spannungen $U_u$, $U_v$, $U_w$ erfassbar. Es gilt:

$$I_u R_u + U_u + \frac{dI_u}{dt}L_u + U_{u,ind} - I_v R_v - U_v - \frac{dI_v}{dt}L_v - U_{v,ind} = 0$$

$$I_v R_v + U_v + \frac{dI_v}{dt}L_v + U_{v,ind} - I_w R_w - U_w - \frac{dI_w}{dt}L_w - U_{w,ind} = 0$$

$$I_w R_w + U_w + \frac{dI_w}{dt}L_w + U_{w,ind} - I_u R_u - U_u - \frac{dI_u}{dt}L_u - U_{u,ind} = 0$$

(Gleichungen 1).

Bei bekannten Induktivitäten $L_u$, $L_v$, $L_w$ und Phasenwiderständen $R_u$, $R_v$, $R_w$ können BEMF-Spannungen $U_{BEMF1}$, $U_{BEMF2}$, $U_{BEMF3}$ wie folgt berechnet werden:

$$U_{BEMF1} = U_{u,ind} - U_{v,ind}$$

$$U_{BEMF2} = U_{v,ind} - U_{w,ind}$$

$$U_{BEMF3} = U_{w,ind} - U_{u,ind}$$

(Gleichungen 2).

Auf Grundlage der BEMF-Spannungen kann eine rotatorische Position (Drehwinkel) des Rotors 240 bezüglich der Rotationsachse 500 berechnet werden. Dazu werden die drei BEMF-Spannungen mittels Clark-Transformation in eine zweidimensionale Darstellung überführt und der Drehwinkel unter Verwendung der Arkustangensfunktion berechnet. Diese Berechnung ist also von $L_u$, $L_v$, $L_w$, $R_u$, $R_v$, $R_w$, $I_u$, $I_v$, $I_w$, $U_u$, $U_v$, $U_w$ abhängig.

Hierbei sind die Spannungs- und Strommessungen mit nur geringen Fehlern behaftet. Die Induktivitäten sind im Beispiel (Luftspulen) näherungsweise temperaturunabhängig und im für die Regelung relevanten Frequenzbereich nahezu konstant. Eine deutliche Temperaturabhängigkeit weist dagegen der Phasenwiderstand R (also $R_u$, $R_v$, $R_w$) auf:

$$R = R_{20}(1 + \alpha_{Cu}\Delta T), \qquad mit\ \alpha_{Cu} = 3.93 \cdot 10^{-3} \ ,$$

(Gleichung 3).

wobei $R_{20}$ der Widerstand bei einer Bezugstemperatur von 20 °C, $\Delta T$ eine Temperaturdifferenz und $\alpha_{Cu}$ ein materialabhängiger Temperaturkoeffizient (hier für Spulenwindungen und Zuleitungsadern aus Kupfer) ist. Beispielsweise ändern 10 K Temperaturunterschied den Widerstand um ca. 4 %. Auch andere Faktoren (z. B. Drift, Korrosion, Bruch einer redundanten Driveline-Ader, thermische Einflüsse) können den Phasenwiderstand beeinflussen, der somit als zeitlich veränderlich anzusehen ist und zum Zweck der Regelung im laufenden Betrieb der Pumpe bestimmt werden soll.

Die Steuereinheit 300 ist dementsprechend eingerichtet zum Bestimmen des jeweiligen Phasenwiderstands mittels der hier vorgeschlagenen Verfahren.

Insbesondere ist die Steuereinheit 300 eingerichtet zum

Erzeugen eines Steuersignals für eine jeweilige der Mehrzahl von Motorspulen 221, wobei das Steuersignal mit einem Modulationssignal beaufschlagt wird,

Demodulieren des Messsignals zum Bestimmen eines veränderlichen charakteristischen elektrischen Widerstands einer elektrischen Anordnung (Phasenwiderstand), umfassend die jeweilige der Mehrzahl von Motorspulen und eine jeweilige Zuleitung zum Verbinden der jeweiligen der Mehrzahl von Motorspulen 221 mit der Steuereinheit 300.

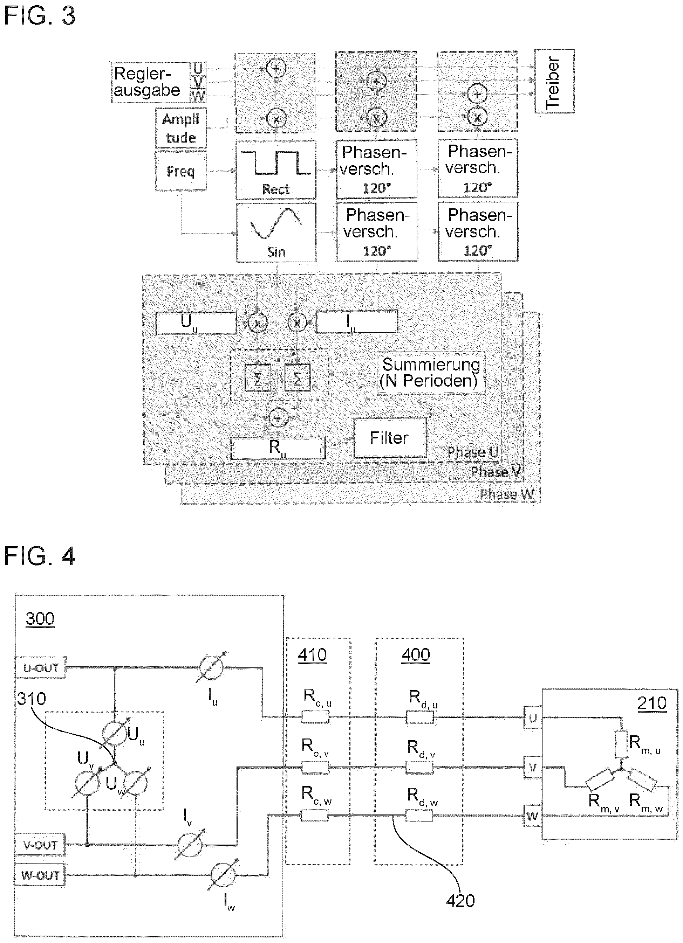

Ein Beispiel des vorbeschriebenen Verfahrens ist illustriert in FIG. 3. Die Bestimmung der Phasenwiderstände ist hierbei als kontinuierliche Hintergrundmessung im Betrieb der Pumpe implementiert. Das Steuersignal entspricht dabei einer jeweiligen Reglerausgabe für die Phasen u, v, w. Die dem Steuersignal entsprechende Spannung wird durch Pulsweitenmodulation (PWM) als PWM-Signal bereitgestellt.

Zur Messung des Phasenwiderstands, $R_p$, zu einer gegebenen Motorphase (p = u, v, w) ist die Steuereinheit 300 dazu eingerichtet, das Steuersignal mit dem Modulationssignal durch Variation der Pulsbreitenmodulation, hier durch Aufaddieren einer Signalform, insbesondere eines Rechtecksignals, auf das PWM-Signal zu beaufschlagen. Dies kann nacheinander für jede Phase oder phasenversetzt (um 120 °) erfolgen, wie in FIG. 3 gezeigt. Mittels eines Treibers werden die entsprechenden Motorspulen 221 mit dem so modifizierten Steuersignal beaufschlagt.

Die Amplitude des Modulationssignals ist dabei variabel und wird vorzugsweise so gewählt, dass Störungen des Motorbetriebs vermieden bzw. minimiert werden (bsp. als kleinste Auflösung der PWM). Die Frequenz des Modulationssignals wird ebenfalls vorzugsweise so gewählt, dass Störungen vermieden werden, beispielsweise im Hinblick auf die Drehzahl der Pumpe und/oder eine Herzfrequenz. Eine Modulationsfrequenz des Modulationssignals kann beispielsweise weniger als eine Drehfelddrehzahl, vorzugsweise weniger als 50 Hz, betragen. Beispielhaft kann etwa eine Frequenz von 10 Hz gewählt werden. Die Drehfelddrehzahl 240 kann beispielweise im Bereich von 0,1 bis 1 kHz liegen.

Die Steuereinheit 300 ist dazu eingerichtet, einen durch das Beaufschlagen des Steuersignals mit dem Modulationssignal verursachten Modulationsanteil des Messsignals mittels Demodulation zu erfassen und aus dem demodulierten Signal den Phasenwiderstand zu bestimmen.

Das Messsignal ($I_p$, $U_p$ mit p = u, v, w) wird dazu im jeweiligen Modulationszustand an dem jeweils mit dem Modulationssignal beaufschlagten Satz von Motorphasen 221 abgegriffen. Die einzelnen Messpunkte des Messsignals können mit dem Modulationssignal oder einem Sinus der jeweiligen Phase gewichtet werden. Zusätzlich oder alternativ kann zur Gewichtung eine Fensterfunktion angewendet werden, um mehr Frequenzanteile für die Strom- und Spannungsmessung zu erfassen, wodurch ein besserer Signal-Rausch-Abstand erzielbar sein kann.

Das (ggf. gewichtete) Messsignal wird über N Perioden summiert (also kumuliert, alternativ gemittelt). Die N Perioden entsprechen dabei beispielsweise einem Intervall von mindestens 1 s und/oder höchstens 10 s. Das Intervall kann wie erwähnt an die momentanen Anforderungen angepasst werden; beispielsweise kann eine schnelle, kurzzeitige Messung mit reduziertem Intervall bei erhöhter Modulationsamplitude durchgeführt werden.

Aus dem kumulierten Signal für die Ströme und Spannungen ($I_p$, $U_p$) erfolgt die Berechnung des Phasenwiderstands. Ein zusätzlicher Filter kann angewandt werden, um die erhaltenen Werte zu glätten oder gestörte Werte zu entfernen.

FIG. 4 illustriert beispielhafte Komponenten des Pumpensystems 100 für das in FIG. 3 illustrierte Verfahren. Motor 210 und Steuereinheit 300 sind mittels der Driveline 400 und einer lösbaren Steckverbindung 410 miteinander verbunden. Die Driveline 400 in Verbindung mit der Steckverbindung 410 umfasst Zuleitungen zum Verbinden jeder der Motorspulen 221 mit der Steuereinheit 300. Jede der Zuleitungen umfasst eine oder mehrere leitende Adern 420 und einen Kontakt, hier einen Steckkontakt der Steckverbindung 410, zum Verbinden der Ader 420 mit der Steuereinheit 300. Die Kontakte können alternativ fest ausgeführt sein. Der Phasenwiderstand jeder Phase p = u, v, w umfasst einen Widerstand $R_{m,p}$ der jeweiligen Motorspule bzw. Motorspulen, einen Widerstand $R_{d,p}$ der jeweiligen Ader bzw. Adern der Driveline und einen Widerstand $R_{c,p}$ des jeweiligen Kontakts. Die Steuereinheit umfasst jeweilige Mittel zum Erfassen der an den jeweiligen Anordnungen abfallenden Spannungen $U_p$ und der dadurch fließenden Ströme $I_p$ (jeweilige Schaltzeichen mit Pfeil im Kreis).

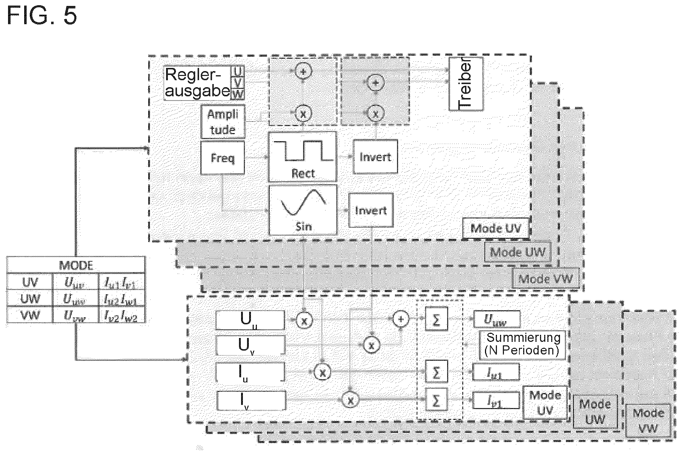

FIG. 5 illustriert ein weiteres Beispiel des vorgeschlagenen Verfahrens. Ausgangspunkt ist die Überlegung, dass bei dem Beispiel nach FIG. 3/4 eine virtuelle Spannungsmessung (mit virtuellem Sternpunkt 310 der Spannungsmessung wie in FIG. 4 gezeigt) erfolgt, die nur dann genau ist, wenn die Phasenwiderstände aller Phasen näherungsweise gleich sind. Eine signifikante Abweichung eines Phasenwiderstands von den übrigen führt zu einer Verfälschung der Werte an den anderen Phasen. Um dennoch eine genaue Messung durchführen zu können, kann vorgesehen werden, die Sternpunkte galvanisch zu verbinden. Da diese Lösung einen zusätzlichen Leiter erfordert, wird die nachfolgend beschriebene alternative Verfahrensvariante bereitgestellt.

Hierbei wird ein differentielles Messverfahren angewandt. Die Modulation findet dabei beispielsweise gegenphasig an zwei Phasen statt, während die dritte Phase nicht angeregt wird. Die Steuereinheit 300 ist also dazu eingerichtet, nacheinander eine Mehrzahl von Modulationszuständen (im Beispiel nach FIG. 5 als Mode uv/uw/vw bezeichnet) vorzugeben, wobei in jedem Modulationszustand ein jeweiliger Satz von Motorphasen 221 gegenphasig oder phasenverschoben mit dem Modulationssignal beaufschlagt wird.

Synchron zum jeweiligen Modulationszustand wird eine Differenz der an den entsprechenden Motorspulen anliegenden Spannungen sowie der jeweiligen Ströme mittels eines Sinusdetektors der Steuereinheit 300 erfasst. Die Messung wird wie oben über N Perioden wiederholt und kumuliert, anschließend wird das Phasenpaar gewechselt. Insgesamt werden so im vorliegenden Beispiel also in drei Messschritten (entsprechend den drei Modulationszuständen) drei Differenzspannungen $U_{uv}$, $U_{uw}$, $U_{vw}$ und sechs Ströme $I_{u1}$, $I_{v1}$, $I_{w1}$, $I_{u2}$, $I_{v2}$, $I_{v2}$ erfasst. Diese erfüllen die folgenden Gleichungen:

$$U_{uv} = R_u \cdot I_{u1} + R_v \cdot I_{v1}$$
$$U_{uw} = R_u \cdot I_{u2} + R_w \cdot I_{w1}$$
$$U_{vw} = R_v \cdot I_{v2} + R_w \cdot I_{w2}$$

(Gleichungen 4).

Dabei ist $U_{uv}$ die erfasste Amplitude der Spannungsdifferenz zwischen Phase u und v, $U_{uw}$ und $U_{vw}$ analog. $I_{u1}$ ist die erfasste Amplitude des Stroms in Phase v während des uv-Modulationszustands, $I_{v1}$, $I_{w1}$, $I_{u2}$, $I_{v2}$, $I_{v2}$ analog.

Hieraus können die Phasenwiderstände der Phasen u, v, w berechnet werden:

$$R_W = \frac{U_{vw} \cdot I_{v1} \cdot I_{u2} + U_{uw} \cdot I_{u1} \cdot I_{v2} - U_{uv} \cdot I_{u2} \cdot I_{v2}}{I_{w1} \cdot I_{u1} \cdot I_{v2} + I_{w2} \cdot I_{v1} \cdot I_{u2}}$$

$$R_v = \frac{U_{vw}}{I_{v2}} - \frac{R_w \cdot I_{w2}}{I_{v2}}$$

$$R_u = \frac{U_{uw}}{I_{u2}} - \frac{R_w \cdot I_{w1}}{I_{u2}}$$

(Gleichungen 5).

Das differentielle Messverfahren nach FIG. 5 zeichnet sich durch eine besonders hohe Genauigkeit aus, da eine Änderung eines Phasenwiderstands wie erwähnt nicht an die Messung der der übrigen Phasenwiderstände gekoppelt ist.

Im Folgenden werden noch einige alternative Varianten der beschriebenen Messverfahren beschrieben. Bei dem vorbeschriebenen differentiellen Verfahren wird jeweils eine (dritte) Motorphase nicht angeregt und dementsprechend kein dazu gehöriges Messsignal erfasst. Es ist zusätzlich möglich, auch die dritte Phase aktiv mit einem Strom zu beaufschlagen und das dem Strom in der dritten Phase entsprechende Messsignal zu erfassen. Dann kann das differentielle Messverfahren mit zwei statt der oben beschriebenen drei Schritte durchgeführt werden, wobei in beiden Schritten die jeweilige dritte Phase mit unterschiedlichen Strömen beaufschlagt wird.

Die Phasenwiderstände können dann auf Grundlage des folgenden Gleichungssystems berechnet werden:

$$\begin{pmatrix} U_{vw1} \\ U_{uw1} \\ U_{vw2} \end{pmatrix} = \begin{pmatrix} R_v & R_w & 0 & 0 \\ -R_u & R_w - R_u & 0 & 0 \\ 0 & 0 & R_v & R_w \end{pmatrix} \begin{pmatrix} I_{v1} \\ I_{w1} \\ I_{v2} \\ I_{w2} \end{pmatrix}$$

(Gleichung 6).

Dabei ist $U_{vw1}$ die erfasste Amplitude der Spannungsdifferenz zwischen Phase v und w während des ersten Messschritts, $U_{uw1}$ und $U_{vw2}$ analog. $I_{w2}$ ist die erfasste Amplitude des Stroms in Phase w während des zweiten Messschritts, $I_{v1}$, $I_{w1}$, $I_{v2}$ analog.

Es ergibt sich für die Phasenwiderstände:

$$R_u = \frac{-I_{v1}I_{w1}U_{vw2} - I_{v1}I_{w2}U_{uw1} + I_{v2}I_{w1}U_{uw1} - I_{v2}I_{w1}U_{vw1}}{I_{v1}^{\,2}I_{w2} - I_{v1}I_{v2}I_{w1} + I_{v1}I_{w1}I_{w2} - I_{w1}^{\,2}I_{v2}}$$

$$R_v = \frac{-I_{w1}U_{uw1} + I_{w2}U_{vw1}}{I_{v1}I_{w2} - I_{v2}I_{w1}}$$

$$R_w = \frac{I_{v1}U_{uw} - I_{v2}U_{vw1}}{I_{v1}I_{w2} - I_{v2}I_{w1}}$$

(Gleichungen 7).

Bei einer Vektorregelung der Drehung des Rotors mit zweidimensionalem dq-System können auch die d- und q-Komponenten direkt moduliert werden. In diesem Fall entsteht ein mit der Drehung gemischtes Messsignal der Spannungsdifferenzen und Ströme. Dieses kann direkt mit entsprechenden Detektoren erfasst und/oder vor dem Erfassen mit der Drehung entmischt werden.

Unabhängig von dem speziell gewählten Messverfahren kann anstelle der erwähnten Rechtecksmodulation auch eine andere Signalform vorgesehen sein. Hier kommen etwa Sinus, Rauschen oder Pseudozufallssequenzen, beispielsweise Gold-Codes, in Frage. Alle Messverfahren sind hier für drei Motorphasen illustriert, lassen sich aber in offensichtlicher Weise auf andere Zahlen von Motorphasen verallgemeinern.

Es kann vorgesehen sein, dass die Steuereinheit 300 zum alternativen Durchführen beider Messverfahren, also der differentiellen Messung entsprechend FIG. 5 und der Einzelphasenmessung nach FIG. 3, eingerichtet ist. Die Steuereinheit 300 kann insbesondere dazu eingerichtet sein, bei einem Ausfall eines Detektors für das Messsignal einer Phase von der differentiellen Messung auf die Einzelphasenmessung zu wechseln. Auf diese Weise ist eine Messung auch bei einem solchen Ausfall weiterhin möglich. Die Steuereinheit kann in diesem Fall eine Warnung ausgeben, so dass die Steuereinheit gewartet oder ausgetauscht werden kann.

Unabhängig von dem speziell gewählten Messverfahren ist die Steuereinheit 300 bevorzugt dazu eingerichtet, die Rotation des Rotors 240 sowie zumindest eine translatorische Position des Rotors 240 (insbesondere entlang der Rotationsachse) basierend auf dem Messsignal und einem Modell wenigstens eines Teils der Rotationsfluidpumpe 200 zu erfassen und zu regeln, wobei das Modell den Phasenwiderstand umfasst. Zusätzlich können translatorische und/oder rotatorische Positionen in weiteren Freiheitsgraden regelbar sein.

Die Steuereinheit 300 ist dazu eingerichtet, die Phasenwiderstände wiederholt für jeweils ein erstes Messintervall durch Demodulieren des mindestens einen Messsignals gemäß den oben beschriebenen Verfahren zu bestimmen. Die Steuereinheit kann ferner dazu eingerichtet sein, eine Änderung des Phasenwiderstandes über ein zweites Messintervall (insbesondere ebenfalls wiederholt für jeweils ein zweites Messintervall) basierend auf einer Temperaturänderung in mindestens einer der Motorspulen zu bestimmen. Das zweite Messintervall ist dabei kürzer als das erste Messintervall. Somit können Messungen über das erste Messintervall durch Demodulieren und über das zweite Messintervall basierend auf der Temperaturänderung miteinander kombiniert werden, wodurch eine Bestimmung des Phasenwiderstands bzw. seiner Änderungen mit guter Genauigkeit über sowohl kürzere als auch längere Zeitskalen möglich ist.

Die Steuereinheit 300 ist zum Zweck der Bestimmung mittels Temperaturänderung eingerichtet zum:

Bestimmen, basierend auf dem mindestens einen Messsignal und einem thermischen Modell, einer Änderung einer Temperatur wenigstens eines Teils der Rotationsfluidpumpe 200, umfassend die mindestens eine der Mehrzahl von Motorspulen 221 (im Folgenden kurz Motortemperatur), über das zweite Messintervall; und

Bestimmen, basierend auf der bestimmten Änderung der Motortemperatur, eines Wertes oder einer Änderung des Phasenwiderstands für die mindestens eine der Mehrzahl von Motorspulen 221.

Somit ist eine schnelle Schätzung des Phasenwiderstands bei Temperaturänderungen aufgrund kurzzeitig eingebrachter Leistung möglich. Eine solche Erwärmung erfolgt in Bruchteilen einer Sekunde, entsprechend kurz wird das zweite Messintervall gewählt.

Als thermisches Modell kann ein einfaches Modell gewählt werden, beispielsweise wie folgt, basierend auf einer thermischen Kapazität $C_{th}$:

$$C_{th}\frac{dT}{dt} = \dot{Q} = P - E$$

(Gleichung 8).

Dabei ist Q die eingebrachte Wärme, P die eingebrachte Leistung, E die an die Umgebung abgegebene Leistung und T die Motortemperatur. Die Steuereinheit 300 ist also dazu eingerichtet sein, die Änderung der Motortemperatur (und damit letztlich die Änderung des Phasenwiderstands) basierend auf einem auf Grundlage des Messsignals bestimmten Leistungseintrag in die Rotationsfluidpumpe 200 und basierend auf einer geschätzten thermischen Leistungsabgabe der Rotationsfluidpumpe 200 zu bestimmen. Die eingebrachte Leistung P kann präzise aufgrund der Strom- und Spannungsmessung berechnet werden. Die abgegebene Leistung E wird dagegen geschätzt, etwa durch folgende Näherung:

$$E = \frac{1}{R_k}(T - T_e)$$

(Gleichung 9).

Dabei ist $R_k$ ein thermischer Kontaktwiderstand (darstellbar in Einheiten von K/W), $T_e$ die Temperatur eines Kontaktmaterials. In einer Variante kann auch die an den Rotor übertragene Leistung in der abgegebenen Leistung E berücksichtigt werden.

Im Betrieb der Pumpe besteht normalerweise ein Gleichgewicht zwischen Motortemperatur und Temperatur des Kontaktmaterials. Wird plötzlich die Leistung P sehr groß, kommt es zu einer schnellen Änderung der Motortemperatur. $T_e$ wird sich demgegenüber in aller Regel deutlich langsamer ändern, im Fall einer implantierten Blutpumpe insbesondere aufgrund der größeren Wärmekapazität und aktive Kühlung durch das Blut. Diese Änderung hängt von zahlreichen Einflussfaktoren (einschließlich Strömungsbedingungen in der Pumpe sowie Blut- und Körpertemperatur) ab und kann daher nicht genau geschätzt werden. In der vorbeschriebenen (langsamen) Bestimmung des Phasenwiderstands über das erste Messintervall durch Modulation ist jedoch näherungsweise die Information über ein mittleres $T_e$ enthalten, da mit dem Widerstand auch T genau bekannt ist. Basierend auf der Annahme, dass $T_e$ konstant ist, kann das Modell der thermischen Kapazität linearisiert werden.

Die Änderung $\Delta R$ des Phasenwiderstands R kann schließlich geschätzt werden als:

$$\Delta R = R_0 \alpha_{Cu} \Delta T, \quad mit \ \Delta T = \frac{R_k}{(1 + R_k C_{th} s)} UI$$

(Gleichung 10).

Dabei ist $R_0$ der elektrische Widerstand bei der Ausgangstemperatur, $\Delta T$ die geschätzte Temperaturdifferenz und $\alpha_{Cu}$ ein materialabhängiger Temperaturkoeffizient wie oben definiert.

Die Messungen über das erste Messintervall durch Demodulieren und über das zweite Messintervall basierend auf der Temperaturänderung können etwa folgendermaßen miteinander kombiniert werden:

$$R = R_s + \Delta R - \Delta R_{lp}$$

(Gleichung 11).

Dabei ist R der Phasenwiderstand, $R_s$ der mittels Modulation/Demodulation ("Hintergrundmessung") geschätzte Wert (bestimmt mit dem langsameren Takt entsprechend dem Intervall $T_l$), $\Delta R$ die aufgrund der Temperaturänderung geschätzte Differenz (bestimmt mit dem schnelleren Takt entsprechend dem Intervall $T_s$, beispielsweise dem Takt der Regelung), $\Delta R_{lp}$ der zwischen dem letzten und vorletzten Update von $R_s$ gemittelte Wert von $\Delta R$.

Gleichung 11 wurde aufgrund der folgenden Überlegungen gewählt. Schnelle Widerstandsänderungen durch Leistungseintrag sollten im Gesamtwiderstand sofort berücksichtigt werden. Allerdings wirkt sich die Erwärmung auch auf die Hintergrundmessung aus. Daher wird bei jedem Update der Mittelwert der durch Leistung erzeugten Widerstandsänderung abgezogen, da dieser in dem neuen Wert $R_s$ der Hintergrundmessung enthalten ist.

Die Steuereinheit 300 kann zusätzlich dazu eingerichtet sein, basierend auf dem Phasenwiderstand einen Verbindungszustand zwischen der Rotationsfluidpumpe 200 und der Steuereinheit 300 und/oder einen Defektzustand der Rotationsfluidpumpe 200 und/oder der Driveline 400 zu erfassen (wie weiter oben beschrieben aufgrund von entsprechenden Änderungen des Phasenwiderstands). Für das Erfassen des Verbindungszustands kann etwa vorgesehen sein, bei getrennter Verbindung eine Widerstandsmessung mit hoher Amplitude und geringer Integrationszeit durchzuführen, wodurch eine schnelle Messung möglich ist. Bei Herstellen der Verbindung (erfassbar als sprunghafte Widerstandsänderung) kann dann entsprechend der obigen Ausführungen zu diesen Parametern die Amplitude gesenkt und die Integrationsdauer erhöht werden. Bei Entfernen der Pumpe wird erneut in den schnellen Modus gewechselt.

Liste der Bezugszeichen:

100     Pumpensystem,
200     Rotationsfluidpumpe,
201     Gehäuse,
202     Fluideinlass,
203     Fluidauslass,
204     Kavität,
210     Motor,
220     Stator,
221     Motorspulen,

240     Rotor,
241     Beschaufelung,
242     Rotormagnetanordnung,
300     Steuereinheit,
310     virtueller Sternpunkt,
400     Driveline,
410     Steckverbindung,
420     leitende Ader,
500     Rotationsachse.

**Patentansprüche**

1.  Steuereinheit (300) für eine Rotationsfluidpumpe (200), insbesondere eine Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221), umfasst, wobei die Steuereinheit (300) eingerichtet ist zum:

    Erzeugen eines Steuersignals für mindestens eine der Mehrzahl von Motorspulen (221), wobei das Steuersignal mit einem Modulationssignal beaufschlagt wird,
    Erfassen mindestens eines Messsignals, entsprechend einem durch die mindestens eine der Mehrzahl von Motorspulen (221) fließenden Strom und/oder einer an der mindestens einen der Mehrzahl von Motorspulen (221) anliegenden Spannung,
    Demodulieren des mindestens einen Messsignals zum Bestimmen mindestens eines veränderlichen charakteristischen elektrischen Widerstands einer elektrischen Anordnung, umfassend die mindestens eine der Mehrzahl von Motorspulen (221) und eine zum Verbinden der mindestens einen der Mehrzahl von Motorspulen (221) mit der Steuereinheit (300) eingerichtete Zuleitung.

2.  Steuereinheit (300) für eine Rotationsfluidpumpe (200), insbesondere eine Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221) umfasst, wobei die Steuereinheit (300) eingerichtet ist zum:

    Erfassen mindestens eines Messsignals, entsprechend einem durch mindestens eine der Mehrzahl von Motorspulen (221) fließenden Strom und/oder einer an der mindestens einen der Mehrzahl von Motorspulen (221) anliegenden Spannung,
    Bestimmen, basierend auf dem mindestens einen Messsignal und einem thermischen Modell, einer Änderung einer Temperatur wenigstens eines Teils der Rotationsfluidpumpe (200), umfassend die mindestens eine der Mehrzahl von Motorspulen (221), über ein Messintervall; und
    Bestimmen, basierend auf der bestimmten Änderung der Temperatur, eines Wertes oder einer Änderung mindestens eines veränderlichen charakteristischen elektrischen Widerstands einer elektrischen Anordnung, umfassend die mindestens eine der Mehrzahl von Motorspulen (221) und eine zum Verbinden der mindestens einen der Mehrzahl von Motorspulen (221) mit der Steuereinheit (300) eingerichtete Zuleitung.

3.  Steuereinheit (300) nach Anspruch 1, ferner eingerichtet zum:

    Bestimmen, basierend auf dem mindestens einen Messsignal und einem thermischen Modell, einer Änderung einer Temperatur wenigstens eines Teils der Rotationsfluidpumpe (200), umfassend die mindestens eine der Mehrzahl von Motorspulen (221), über ein Messintervall; und
    Bestimmen, basierend auf der bestimmten Änderung der Temperatur, eines Wertes oder einer Änderung des mindestens einen veränderlichen charakteristischen elektrischen Widerstands.

4.  Steuereinheit (300) nach Anspruch 3, dazu eingerichtet,

    den mindestens einen veränderlichen charakteristischen elektrischen Widerstand in einem Normalbetrieb der Rotationsfluidpumpe (200) wiederholt für jeweils ein erstes Messintervall durch Demodulieren des mindestens einen Messsignals zu bestimmen, und
    eine Änderung des mindestens einen veränderlichen charakteristischen elektrischen Widerstands über ein zweites Messintervall, das kürzer ist als das erste Messintervall, basierend auf der bestimmten Änderung der Temperatur zu bestimmen, insbesondere in Antwort auf ein Erfassen eines gegenüber dem Normalbetrieb erhöhten Leistungseintrags in die Rotationsfluidpumpe (200).

5. Steuereinheit (300) nach einem der Ansprüche 1 oder 3 bis 4, wobei der Stator (220) eine Mehrzahl von Motorphasen, umfassend jeweils mindestens eine der Mehrzahl von Motorspulen (221), umfasst und die Steuereinheit (300) dazu eingerichtet ist,

nacheinander eine Mehrzahl von Modulationszuständen vorzugeben, wobei in jedem Modulationszustand ein jeweiliger Satz von Motorphasen gegenphasig oder phasenverschoben mit dem Modulationssignal beaufschlagt wird und
wobei das Messsignal im jeweiligen Modulationszustand an dem jeweils mit dem Modulationssignal beaufschlagten Satz von Motorphasen abgegriffen wird.

6. Steuereinheit (300) nach einem der Ansprüche 1 oder 3 bis 5, wobei eine Modulationsfrequenz des Modulationssignals weniger als eine Drehfelddrehzahl eines die Rotation des Rotors (240) verursachenden Drehfelds, vorzugsweise weniger als 50 Hz, beträgt und/oder wobei zumindest ein Anteil des erfassten Messsignals und/oder eine basierend auf dem Messsignal bestimmte Größe zum Bestimmen des mindestens einen veränderlichen charakteristischen elektrischen Widerstands zeitlich gemittelt und/oder kumuliert wird, insbesondere über ein Intervall von mindestens 1 s und/oder höchstens 10 s.

7. Steuereinheit (300) nach einem der Ansprüche 1 oder 3 bis 6, eingerichtet dazu, das Steuersignal unter Verwendung von Pulsbreitenmodulation zu erzeugen und mit dem Modulationssignal durch Variation der Pulsbreitenmodulation, insbesondere durch Aufaddieren einer Signalform, insbesondere eines Rechtecksignals, zu beaufschlagen, und/oder dazu, einen durch das Beaufschlagen des Steuersignals mit dem Modulationssignal verursachten Modulationsanteil des Messsignals mittels Synchrondemodulation zu erfassen.

8. Steuereinheit (300) nach Anspruch 2, 3 oder einem der Ansprüche 4 bis 7, soweit er auf Anspruch 2 zurückbezogen ist, dazu eingerichtet, die Änderung der Temperatur basierend auf einem auf Grundlage des Messsignals bestimmten Leistungseintrag in die Rotationsfluidpumpe (200) und/oder basierend auf einer geschätzten thermischen Leistungsabgabe der Rotationsfluidpumpe (200) zu bestimmen.

9. Steuereinheit (300) nach einem der vorhergehenden Ansprüche, ferner dazu eingerichtet, basierend auf dem mindestens einen veränderlichen charakteristischen elektrischen Widerstand einen Verbindungszustand zwischen der Rotationsfluidpumpe (200) und der Steuereinheit (300) und/oder einen Defektzustand der Rotationsfluidpumpe (200) und/oder der Zuleitung zu erfassen.

10. Pumpensystem (100), umfassend

eine Rotationsfluidpumpe (200), umfassend einen zum Fördern von Fluid um eine Rotationsachse (500) rotierbaren Rotor (240) sowie einen Stator (220) mit einer Mehrzahl von Motorspulen (221), und
eine Steuereinheit (300) nach einem der vorhergehenden Ansprüche.

11. Verfahren zum Bestimmen mindestens eines veränderlichen charakteristischen elektrischen Widerstands einer elektrischen Anordnung einer Rotationsfluidpumpe (200), insbesondere einer Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221) umfasst, wobei das Verfahren umfasst:

Erzeugen eines Steuersignals für mindestens eine der Mehrzahl von Motorspulen (221), wobei das Steuersignal mit einem Modulationssignal beaufschlagt wird,
Erfassen mindestens eines Messsignals, entsprechend einem durch die mindestens eine der Mehrzahl von Motorspulen (221) fließenden Strom und/oder einer an der mindestens einen der Mehrzahl von Motorspulen (221) anliegenden Spannung,
Demodulieren des mindestens einen Messsignals zum Bestimmen des mindestens einen veränderlichen charakteristischen elektrischen Widerstands der elektrischen Anordnung, wobei die elektrische Anordnung die mindestens eine der Mehrzahl von Motorspulen (221) und eine zum Verbinden der mindestens einen der Mehrzahl von Motorspulen (221) mit der Steuereinheit (300) eingerichtete Zuleitung umfasst.

12. Verfahren zum Bestimmen mindestens eines veränderlichen charakteristischen elektrischen Widerstands einer elektrischen Anordnung einer Rotationsfluidpumpe (200), insbesondere einer Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221) umfasst, wobei das Verfahren umfasst:

Erfassen mindestens eines Messsignals, entsprechend einem durch die mindestens eine der Mehrzahl von Motorspulen (221) fließenden Strom und/oder einer an der mindestens einen der Mehrzahl von Motorspulen (221) anliegenden Spannung,

Bestimmen, basierend auf dem mindestens einen Messsignal und einem thermischen Modell, einer Änderung einer Temperatur wenigstens eines Teils der Rotationsfluidpumpe (200), umfassend die mindestens eine der Mehrzahl von Motorspulen (221), über ein Messintervall; und

Bestimmen, basierend auf der bestimmten Änderung der Temperatur, eines Wertes oder einer Änderung des mindestens einen veränderlichen charakteristischen elektrischen Widerstands der elektrischen Anordnung, umfassend die mindestens eine der Mehrzahl von Motorspulen (221) und eine zum Verbinden der mindestens einen der Mehrzahl von Motorspulen (221) mit der Steuereinheit (300) eingerichtete Zuleitung.

13. Verfahren nach Anspruch 11, ferner umfassend:

Bestimmen, basierend auf dem mindestens einen Messsignal und einem thermischen Modell, einer Änderung einer Temperatur wenigstens eines Teils der Rotationsfluidpumpe (200), umfassend die mindestens eine der Mehrzahl von Motorspulen (221), über ein Messintervall; und

Bestimmen, basierend auf der bestimmten Änderung der Temperatur, eines Wertes oder einer Änderung des mindestens einen veränderlichen charakteristischen elektrischen Widerstands.

# FIG. 1

# FIG. 2

## FIG. 3

## FIG. 4

FIG. 5

Europäisches Patentamt
European Patent Office
Office européen des brevets

# EUROPÄISCHER RECHERCHENBERICHT

**Nummer der Anmeldung**

EP 24 20 1968

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | HANG JUN ET AL: "A signal injection method for fault diagnosis of high-resistance connection in vector-controlled PMSM drive system", IECON 2017 - 43RD ANNUAL CONFERENCE OF THE IEEE INDUSTRIAL ELECTRONICS SOCIETY, IEEE, 29. Oktober 2017 (2017-10-29), Seiten 5021-5026, XP033280291, DOI: 10.1109/IECON.2017.8216867 [gefunden am 2017-12-15] | 1,5-7, 9-11 | INV. H02P21/14 H02P23/14 H02P29/024 H02P29/60 A61M60/538 ADD. H02P6/182 |
| Y | * Zusammenfassung * | 3,13 | |
| A | * II. PMSM Model, III. Fault Diagnosis; Abbildungen 2-3 * | 4 | |
| | ----- | | |
| X | DE LA BARRERA PABLO M ET AL: "Online Voltage Sensorless High-Resistance Connection Diagnosis in Induction Motor Drives", IEEE TRANSACTIONS ON INDUSTRIAL ELECTRONICS, IEEE SERVICE CENTER, PISCATAWAY, NJ, USA, Bd. 62, Nr. 7, 1. Juli 2015 (2015-07-01), Seiten 4374-4384, XP011581662, ISSN: 0278-0046, DOI: 10.1109/TIE.2014.2385038 [gefunden am 2015-05-15] | 1,5-7, 9-11 | RECHERCHIERTE SACHGEBIETE (IPC) H02P A61M |
| Y | * Zusammenfassung * | 3,13 | |
| A | * I. Introduction, II.High-Resistance Connection Diagnosis Method; Abbildungen 2,5 * | 4 | |
| | ----- | | |
| Y | US 2002/113615 A1 (ATARASHI HIROFUMI [JP]) 22. August 2002 (2002-08-22) | 2,3,8, 10,12,13 | |
| A | * Absatz [0096] - Absatz [0109]; Abbildungen 1-3 * * Absatz [0111] - Absatz [0149]; Abbildungen 4-5B * | 4 | |
| | ----- | | |
| | -/-- | | |

Der vorliegende Recherchenbericht wurde für alle Patentansprüche erstellt

| Recherchenort | Abschlußdatum der Recherche | Prüfer |
|---|---|---|
| München | 5. März 2025 | Landi, Matteo |

EPO FORM 1503 03.82 (P04C03)

Europäisches Patentamt

European Patent Office

Office européen des brevets

**EUROPÄISCHER RECHERCHENBERICHT**

**Nummer der Anmeldung**

EP 24 20 1968

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2011/084638 A1 (PATEL NITINKUMAR R [US] ET AL) 14. April 2011 (2011-04-14) | 2,3,8, 10,12,13 | |
| A | * Absatz [0071] - Absatz [0079]; Abbildung 1 * | 4 | |
| | ----- | | |
| A | US 2011/050141 A1 (YEH CHIA-CHOU [US] ET AL) 3. März 2011 (2011-03-03) * Absatz [0043] - Absatz [0054]; Abbildungen 1-2 * | 1-13 | |
| | ----- | | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 5. März 2025 | Landi, Matteo |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................

& : Mitglied der gleichen Patentfamilie, übereinstimmendes Dokument

EPO FORM 1503 03.82 (P04C03)

**EP 4 716 091 A1**

**ANHANG ZUM EUROPÄISCHEN RECHERCHENBERICHT
ÜBER DIE EUROPÄISCHE PATENTANMELDUNG NR.**                    EP 24 20 1968

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-03-2025

| Im Recherchenbericht angeführtes Patentdokument | Datum der Veröffentlichung | Mitglied(er) der Patentfamilie | Datum der Veröffentlichung |
|---|---|---|---|
| US 2002113615 A1 | 22-08-2002 | EP 1220439 A2 | 03-07-2002 |
| | | JP 3502040 B2 | 02-03-2004 |
| | | JP 2002199776 A | 12-07-2002 |
| | | US 2002113615 A1 | 22-08-2002 |
| US 2011084638 A1 | 14-04-2011 | CN 102045021 A | 04-05-2011 |
| | | DE 102010041944 A1 | 19-05-2011 |
| | | US 2011084638 A1 | 14-04-2011 |
| US 2011050141 A1 | 03-03-2011 | CN 102004008 A | 06-04-2011 |
| | | DE 102010038560 A1 | 14-04-2011 |
| | | US 2011050141 A1 | 03-03-2011 |

EPO FORM P0461

Für nähere Einzelheiten zu diesem Anhang : siehe Amtsblatt des Europäischen Patentamts, Nr.12/82

20